# EUROPEAN PATENT SPECIFICATION

(11) **EP 3 685 754 B1**
(45) Date of publication and mention of the grant of the patent: **31.05.2023**
(21) Application number: 20153589.5
(22) Date of filing: 24.01.2020
(51) Int. Cl.: A61B 8/00

(54) **ULTRASONIC DIAGNOSTIC APPARATUS**
ULTRASCHALLDIAGNOSEVORRICHTUNG
APPAREIL DE DIAGNOSTIC ULTRASONIQUE

(30) Priority: 24.01.2019 KR 20190009093
(43) Date of publication of application: 29.07.2020
(73) Proprietor: Samsung Medison Co., Ltd., Hongcheon-gun, Gangwon-do, 25108 (KR)
(72) Inventor: KIM, Jongmoon, 01320 Seoul (KR); LEE, Sangmok, 01784 Seoul (KR)
(74) Representative: Arnold & Siedsma

(56) References cited:
- US-A1- 2013 197 364
- US-A1- 2015 105 660
- US-A1- 2018 028 160
- CAROLYN COFFIN: "Work-related musculoskeletal disorders in sonographers: a review of causes and types of injury and best practices for reducing injury risk", REPORTS IN MEDICAL IMAGING, 1 February 2014 (2014-02-01), page 15, XP055698021, DOI: 10.2147/RMI.S34724

## Description

### TECHNICAL FIELD

Embodiments of the disclosure relate to an ultrasonic diagnostic apparatus, and more particularly, to an ultrasonic diagnostic apparatus that can provide convenience to a user by interlocking and controlling different inputters of the ultrasonic diagnostic apparatus.

### BACKGROUND

An ultrasonic diagnostic apparatus irradiates ultrasonic waves to an object and detects echo signals reflected from the object to generate images of a target part inside the object such as tomographic or blood flow of soft tissues, thereby providing information of a required target part.

To operate the ultrasonic diagnostic apparatus, the user can use a control panel and a pedal. At this time, the user may operate the control panel and the pedal by using the user's hands and feet, respectively. In order to set them according to a physical condition of the user, positions of the control panel and the pedal must be adjusted separately.

US2015/105660 describes a cart for an ultrasonic diagnostic apparatus, having a high degree of flexibility in moving a top board on which the ultrasonic diagnostic apparatus is placed, and enabling a stable operation wherever the top board is moved. In the cart for the ultrasonic diagnostic apparatus, a vertical movement mechanism is fixed on a base. A movable part of the vertical movement mechanism is provided with a swing mechanism that swings in the up-and-down direction, enabling up-and-down movement of the top board according to both the vertical movement mechanism and the swing mechanism. The position of the movable part with which the swing mechanism is coupled is displaced forward from the vertical central axis of the vertical movement mechanism. The top board for placing the ultrasonic diagnostic apparatus is coupled with the swinging end of the swing mechanism, via a mechanism for horizontal movement and/or a mechanism for rotating the top board.

US2013/197364 describes an ultrasound diagnosis apparatus which includes a main body supporting ultrasound equipment that generates ultrasound image signals. Additionally, a first and second display unit are electrically coupled to the ultrasound equipment, and display ultrasound images corresponding to the ultrasound image signals. A first coupling unit couples the second display unit and the main body and enables movement of the second display unit relative to the main body. As the position of the second display unit is adjustable, a patient may easily view ultrasound images displayed on the second display unit.

As described above, when the positions of the control panel and the pedal are separately adjusted, problems such as trouble and time wasted due to the adjustment occur. The disclosure has been made to solve the above-described problem, to solve the adjustment of the control panel and the pedal at one time.

### SUMMARY

Therefore, it is an aspect of the disclosure to provide an ultrasonic diagnostic apparatus capable of simultaneously adjusting a control panel and a footrest.

Additional aspects of the disclosure will be set forth in part in the description which follows and, in part, will be obvious from the description, or may be learned by practice of the disclosure.

The ultrasonic apparatus comprises a control panel configured to receive a user input for operating the ultrasonic diagnostic apparatus, and to move a position of the control panel with respect to the ultrasonic diagnostic apparatus;a footrest moving device provided below the ultrasonic diagnostic apparatus, configured to move a footrest provided to mount a user's foot and the ultrasonic diagnostic apparatus with respect to the ground. The footrest moving device comprises a first support configured to support the ultrasonic apparatus on a lower front of the ultrasonic diagnostic apparatus; and a second support configured to support the ultrasonic apparatus on a lower rear of the ultrasonic diagnostic apparatus. The first and second support are configured to be each changed in height.

The ultrasonic diagnostic apparatus comprises a controller configured to control the movement of the control panel and the footrest moving device to interlock.

The controller may interlock so that the footrest moves downward when the control panel moves upward, and may interlock so that the footrest moves upward when the control panel moves downward.

The controller may interlock so that the footrest moves upward when the control panel moves upward, and may interlock so that the footrest moves upward when the control panel moves downward.

The controller may interlock so that the footrest moves backward when the control panel moves forward, and may interlock so that the footrest moves forward when the control panel moves backward.

The controller may interlock so that the footrest moves forward when the control panel moves forward, and may interlock so that the footrest moves backward when the control panel moves backward.

The controller may interlock so that the footrest moves clockwise when the control panel moves counterclockwise, and to interlock so that the footrest moves counterclockwise when the control panel moves clockwise.

The controller may interlock so that the footrest moves counterclockwise when the control panel moves counterclockwise, and may interlock so that the footrest moves clockwise when the control panel moves clockwise.

The ultrasonic diagnostic apparatus may further include a memory configured to store mode settings of the interlocking.

The ultrasonic diagnostic apparatus may further include an inputter configured to receive an input for operating the interlocking of the control panel and the footrest. The inputter may be positioned at any one of a main body of the ultrasonic diagnostic apparatus and the control panel.

The inputter may include a mode change button, a first move button, and a second move button configured to move in a direction opposite to a movement by the first move button.

The control panel may be changed in position by upward and downward movement, left and right movement, forward and backward movement, rotational movement, and tilting.

The footrest moving device may include a first support configured to support the ultrasonic diagnostic apparatus; and a second support configured to support the ultrasonic diagnostic apparatus in a different column than the first support. The footrest moving device may be changed in position by upward and downward movement, left and right movement, forward and backward movement, rotational movement, and tilting. The tilting may be performed by upward and downward movement of the first support.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a view illustrating an external configuration of an ultrasonic diagnostic apparatus according to embodiments of the disclosure;
FIG. 2 is a control block diagram of an ultrasonic diagnostic apparatus according to embodiments of the disclosure;
FIG. 3 is a view illustrating a configuration of an inputter provided in an ultrasonic diagnostic apparatus according to embodiments of the disclosure; and
FIGS. 4 to 9 are views illustrating an interlocking movement of an ultrasonic diagnostic apparatus according to embodiments of the disclosure.

### DETAILED DESCRIPTION

Like reference numerals refer to like elements throughout the specification. Not all elements of embodiments of the disclosure will be described, and description of what are commonly known in the art or what overlap each other in the embodiments will be omitted. The terms as used throughout the specification, such as "~ part," "~ module," "~ member," "~ block," etc., may be implemented in software and/or hardware, and a plurality of "∼ parts," "∼ modules," "∼ members," or "∼ blocks" may be implemented in a single element, or a single "∼ part," "~ module," "∼ member," or "∼ block" may include a plurality of elements.

It will be understood that when an element is referred to as being "connected" to another element, it can be directly or indirectly connected to the other element, wherein the indirect connection includes "connection" via a wireless communication network.

Also, when a part "includes" or "comprises" an element, unless there is a particular description contrary thereto, the part may further include other elements, not excluding the other elements.

Further, when it is stated that a layer is "on" another layer or substrate, the layer may be directly on another layer or substrate or a third layer may be disposed therebetween.

It will be understood that, although the terms first, second, third, etc., may be used herein to describe various elements, it should not be limited by these terms. These terms are only used to distinguish one element from another element.

As used herein, the singular forms "a," "an" and "the" are intended to include the plural forms as well, unless the context clearly indicates otherwise.

An identification code is used for the convenience of the description but is not intended to illustrate the order of each step. Each of the steps may be implemented in an order different from the illustrated order unless the context clearly indicates otherwise.

Prior to the description of the specification, some terms used in the specification will be clarified.

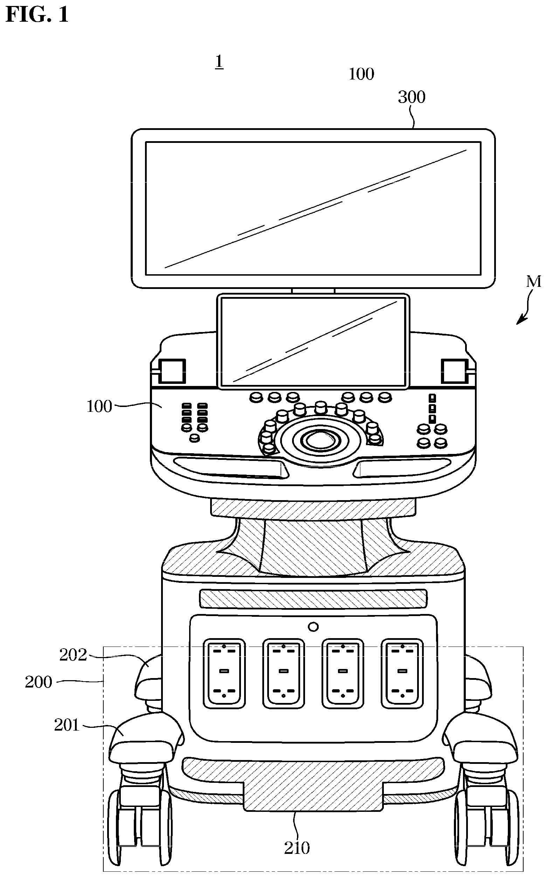

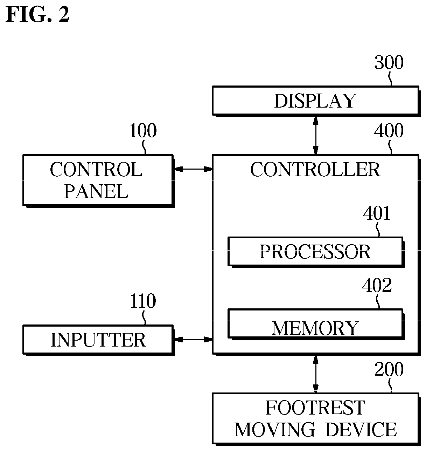

FIG. 1 is a view illustrating an external configuration of an ultrasonic diagnostic apparatus according to embodiments of the disclosure, and FIG. 2 is a control block diagram of an ultrasonic diagnostic apparatus according to embodiments of the disclosure.

Referring to FIG. 1, an ultrasound diagnostic apparatus 1 may include a main body M, a control panel 100, a footrest moving device 200, and a display 300.

The main body M may be connected to an ultrasonic probe (not shown) through a wireless or wired communication network to receive various signals necessary for controlling the ultrasonic probe or to receive analog signals or digital signals corresponding to echo ultrasonic signals transmitted by the ultrasonic probe.

On the other hand, the wireless communication network may refer to a communication network that can transmit and receive signals wirelessly, the main body M may perform wireless communication with the ultrasonic probe or various auxiliary devices through at least one of a short-range communication module and a mobile communication module.

The short-range communication module may refer to a module for short range communication within a predetermined distance. For example, short-range communication technologies may include wireless local access network (WLAN), Wi-Fi, Bluetooth, Zigbee, Wi-Fi Direct (WFD), Ultra-Wideband (UWB), infrared Data Association (IrDA), Bluetooth Low Energy (BLE), Near Field Communication (NFC), and the like, but are not limited thereto.

In addition, the main body M may exchange data with the ultrasonic probe through the wired communication network. The wired communication network may refer to a communication network that can transmit and receive signals by wire.

Meanwhile, the main body M may include the display 300 and the control panel 100. The display 300 and the control panel 100 may receive not only setting information regarding the ultrasonic probe, but also various control commands for controlling the various ultrasonic diagnostic apparatuses 1 from the user. In addition, the main body M may be provided with an inputter 110 to receive an interlocking control command to be described later.

The control panel 100 may refer to a control device that receives various commands from the user. The control panel 100 may be implemented by a button or a keyboard method. For example, the keyboard may be implemented in hardware. The keyboard may include at least one of a switch, a key, a joystick, and a trackball. As another example, the keyboard may be implemented in software such as a graphical user interface. In this case, the keyboard may be displayed through the display 300.

The control panel 100 may be moved relative to the main body M by having a separate connection member. For example, the connection member may be various connection movement parts such as a joint, such as a slide or a roller, so that the control panel 100 may move in various directions with respect to the main body M.

The control panel 100 may be moved in various directions with respect to the main body M according to a user's control command.

For example, the control panel 100 may move forward or backward relative to the ultrasonic diagnostic apparatus 1 so as to be closer or further away from the user using the ultrasonic diagnostic apparatus 1. In addition, the control panel 100 may move in upward or downward direction so as to meet an operation reference of the user using the ultrasonic diagnostic apparatus 1, and may move in the left or right direction with respect to the main body M.

In addition, the control panel 100 may be tilted to adjust an inclination and may be provided to be rotatable with respect to the main body M.

The footrest moving device 200 is provided below the ultrasonic diagnostic apparatus 1 and includes a footrest 210 provided to mount the user's foot, a first support 201 and a second support 202 for moving the ultrasonic diagnostic apparatus 1 with respect to a ground.

The footrest 210 may represent a space provided to mount the user's foot who performs an ultrasound diagnosis. Here, the footrest 210 may be implemented as a foot switch or a foot pedal, in addition to being simply implemented as a support provided to mount the user's foot, thereby controlling various operations of the ultrasonic diagnostic apparatus 1.

The first support 201 and the second support 202 are provided on the lower front and lower rear surfaces of the ultrasonic diagnostic apparatus 1, respectively, to support the ultrasonic diagnostic apparatus 1. When the first support 201 and the second support 202 moves upward or downward at the same height, the height of the entire ultrasonic diagnostic apparatus 1 may be adjusted, and thus the height of the footrest 210 may be adjusted.

When only one of the first support 201 and the second support 202 is changed in height, the inclination of the footrest 210 is adjusted, so that the footrest moving device 200 may perform a tilting operation.

On the other hand, the footrest moving device 200 may move forward or backward relative to the ultrasonic diagnostic apparatus 1 so as to be closer or further away from the user's foot using the ultrasonic diagnostic apparatus 1. In addition, the footrest moving device 200 may move in the upward or downward direction so as to meet the operation reference of the user using the ultrasonic diagnostic apparatus 1, and may move in the left or right direction with respect to the main body M.

In addition, as described above, the footrest moving device 200 may be tilted to adjust the inclination of the footrest 210 and may be provided to be rotatable with respect to the main body M.

The display 300 may display an ultrasonic image of a target part of the object Ob. The ultrasonic image displayed on the display 300 may be a 2D ultrasonic image or a 3D ultrasonic image. The display 300 may display various ultrasonic images according to operation modes of the ultrasonic diagnostic apparatus 1.

When the display 300 is implemented as a touch screen type, the display 300 may also perform a function of the control panel 100. That is, the main body M may receive various commands from the user through at least one of the display 300 and the control panel 100.

The display 300 may be implemented as a Cathode Ray Tube (CRT), a Liquid Crystal Display (LCD), a Light Emitting Diode (LED) display, a Plasma Display Panel (PDP) display, an Organic Light Emitting Diode (OLED) display, etc., which are well-known in the art.

On the other hand, the display 300 may be provided with the connection member like the control panel 200 described above to perform a movement operation in various directions.

The controller 400 may control the ultrasonic diagnostic apparatus 1 and control the control panel 200 and the footrest moving device 200 to interlock with each other.

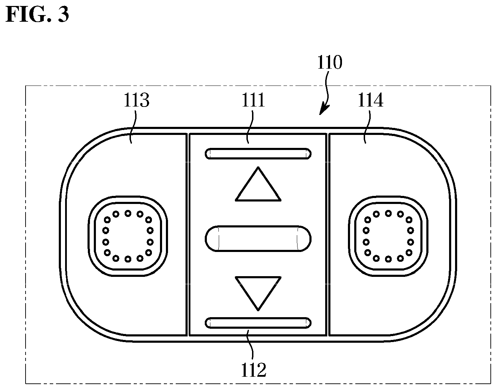

When the controller 400 receives the control command through the inputter 110, the controller 400 may control the control panel 100 and the footrest moving device 200 to operate in interlocking with each other. The inputter 110 may be a device to which the user inputs the operation command for arranging various operator of the ultrasonic diagnostic apparatus 1 to suit the user's body structure. A detailed description of the inputter 110 will be described below with reference to FIG. 3.

The controller 400 may include a processor 401, and may include a ROM in which a control program for controlling the ultrasonic diagnostic apparatus 1 is stored and a RAM used a storage area corresponding to various operations performed in the ultrasound imaging apparatus 1. Also, the controller 400 may be implemented as a graphic processing board including the processor, the RAM, or the ROM on a circuit board, and the processor 401, the RAM, and the ROM may be interconnected through an internal bus.

The controller 400 may include at least one memory 402 for memorizing / storing programs and data, and at least one processor 401 for processing data memorized / stored in the memory 402 according to a program memorized / stored in the memory 402. The controller 400 may include hardware such as the processor 401 and the memory 402, and software such as programs and data memorized / stored in the memory 402.

The memory 402 may store programs and data for controlling the operation of the ultrasonic diagnostic apparatus 1. Particularly, the memory 402 may store instructions executed by the processor 401 and data processed by the instructions. For example, the memory 402 may store data regarding various mode settings of interlocking control between the control panel 100 and the footrest moving device 200. The mode setting refers to the combination of movement between the control panel 100 and the footrest moving device 200. For example, one of the mode settings may be data about interlocking control such that the footrest 210 moves downward when the control panel 100 moves upward and the footrest 210 moves upward when the control panel 100 moves downward.

The memory 402 may include a non-volatile memory, such as read only memory (ROM) and flash memory for storing data for a long time, and a volatile memory, such as static random access memory (S-RAM) and dynamic random access memory (D-RAM) for temporarily storing data.

FIG. 3 is a view illustrating a configuration of an inputter provided in an ultrasonic diagnostic apparatus according to embodiments of the disclosure.

The inputter 110 may refer to an interface device for the user to receive a command to move a position of control panel 100 and footrest moving device 200 of ultrasonic diagnostic apparatus 1. In FIG. 3, the inputter 110 of a simple button form is illustrated, but this is merely for convenience of description, and may be implemented using the switch, a knob, a jog dial, and the like, even if it is not in the button form. In addition, the arrangement and order of the buttons of FIG. 3 are only an example, and may be variously implemented according to a setting or an early production process.

The inputter 110 may be provided in the main body M of the ultrasonic diagnostic apparatus 1 or may be provided in the control panel 100. The position of the inputter 110 is merely an example and may be provided at various positions for easy access for the user to input, and may be implemented by a wired / wireless apparatus separately from the ultrasonic diagnostic apparatus 1.

The interlocking between the control panel 100 and the footrest moving device 200 may be implemented according to various modes. The interlocking between the control panel 100 and the footrest moving device 200 is a combination of respective movement directions or movement methods. When each of the control panel 100 and the footrest moving device 200 can be moved in six directions, 36 combinations may be generated as 6 × 6. However, as described above, the control panel 100 and the footrest moving device 200 may follow a moving method such as tilting or rotating, and the combination thereof may be more various.

The inputter 110 may include a first move button 111, a second move button 112, a mode change button 113, and a stop button 114. The first move button 111 may be a button for allowing the control panel 100 and the footrest moving device 200 to simultaneously move in either direction. The second move button 112 may be a button that moves to be opposite to the first move button 111, that is, the button to move in the opposite direction of the one direction.

The mode change button 113 may refer to a button for changing the movement of the control panel 100 and the footrest moving device 200 by the first move button 111 and the second move button 112 to a mode moving in different directions or in different methods. The various modes may be stored in the above-described memory 402 according to the user's setting. In addition, the memory 402 may store position information of the control panel 100 and the footrest 210 according to the user's body.

The stop button 114 may be a button for stopping the movement of the control panel 100 and the footrest moving device 200 by the first move button 111 and the second move button 112. The control panel 100 and the footrest moving device 200 may perform the movement operation unless there is a separate stop command once a movement command is detected according to a Free / Stop method. The user may confirm that the control panel 100 and the footrest moving device 200 are moved to suit the user's body through the stop button 114 and may input the stop command through the stop button 114.

In addition, the user may adjust a degree of movement of the control panel 100 and the footrest moving device 200 through buttons provided in the inputter 110.

In addition, the above-mentioned description described the configuration of the ultrasonic diagnostic apparatus 1, and the function of the configuration. Hereinafter, various combinations of movements of the control panel 100 and the footrest moving device 200, that is, various interlocking modes will be described in detail.

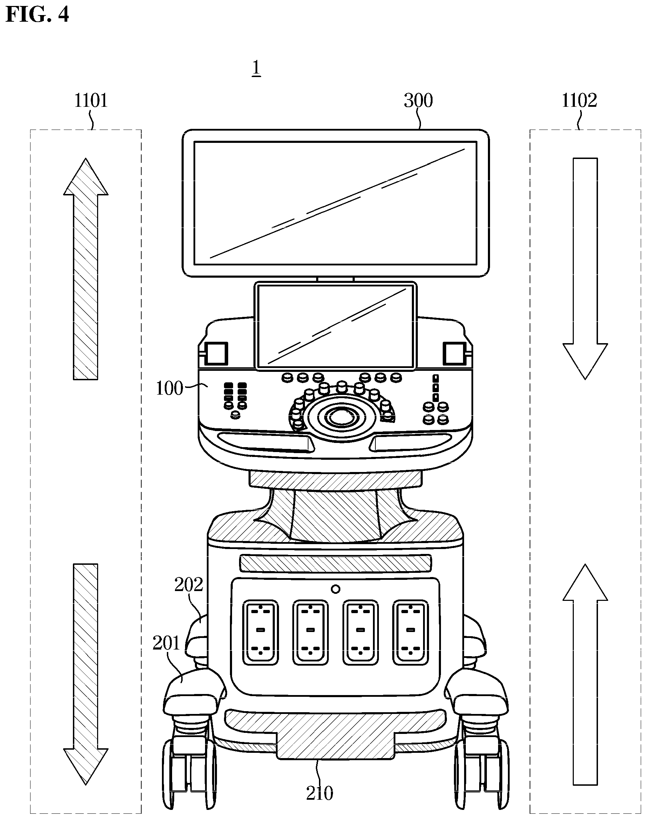

FIG. 4 illustrates a first mode of interlocking control of the controller 400. The controller 400 may control the footrest 210 to move downward when the control panel 100 moves upward. At this time, a distance between the control panel 100 and the footrest moving device 200 moves away faster than moving independently. In contrast, the controller 400 may control the footrest 210 to move upward when the control panel 100 moves downward. At this time, the distance between the control panel 100 and the footrest moving device 200 moves closer together than moving independently. The position of the footrest 210 may be moved together as the footrest moving device 200 is moved. The ultrasonic diagnostic apparatus 1 may generate a larger displacement value between the control panel 100 and the footrest moving device 200 than a conventional independent movement, and thus may quickly provide a suitable state suitable for the user's body.

In addition, the inclination of the footrest 210 may be changed as the footrest moving device 200 performs the tilting operation. For example, the inclination of the footrest 210 may change as only one of the first support 201 or the second support 202 is moved.

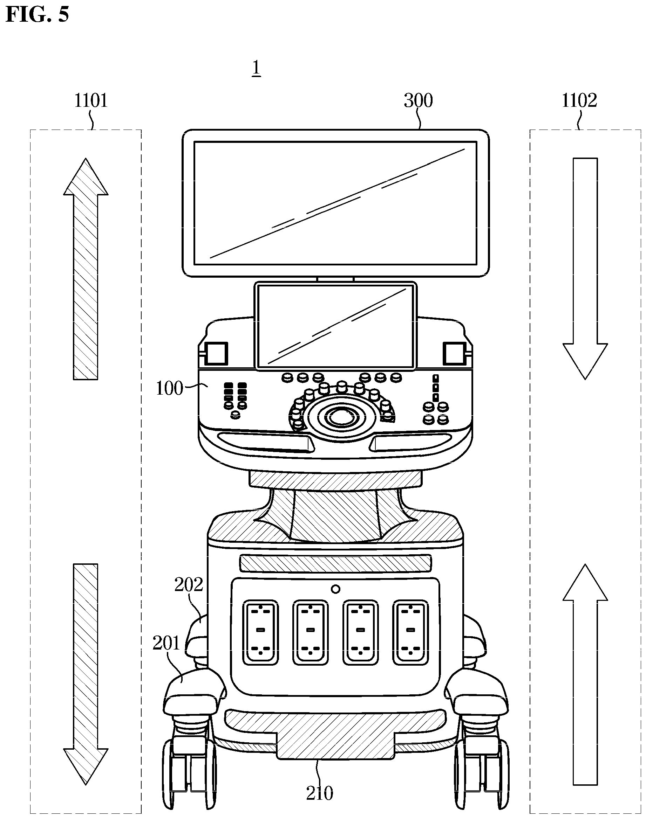

FIG. 5 illustrates a second mode of interlocking control of the controller 400. The controller 400 may control the footrest moving device 200 to move upward simultaneously when the control panel 100 moves upward. At this time, since the height of the control panel 100 changes as the footrest moving device 200 rises, the control panel 100 may be moved to a desired position faster than before. On the contrary, the controller 400 may control the footrest moving device 200 to move downward at the same time when the control panel 100 moves downward.

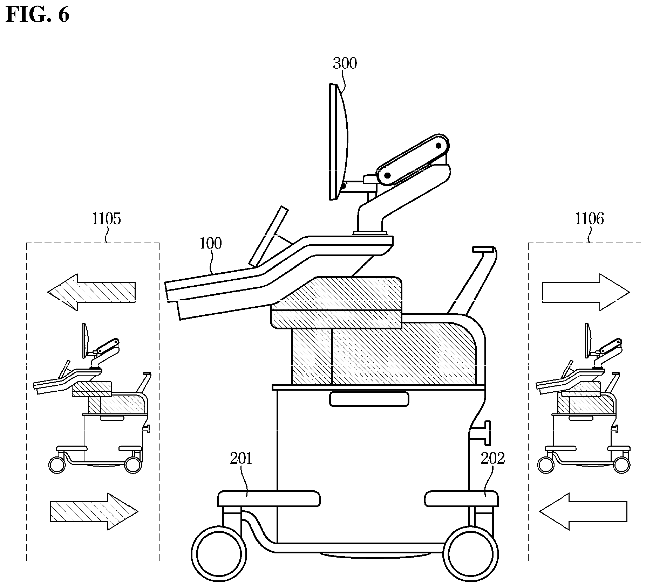

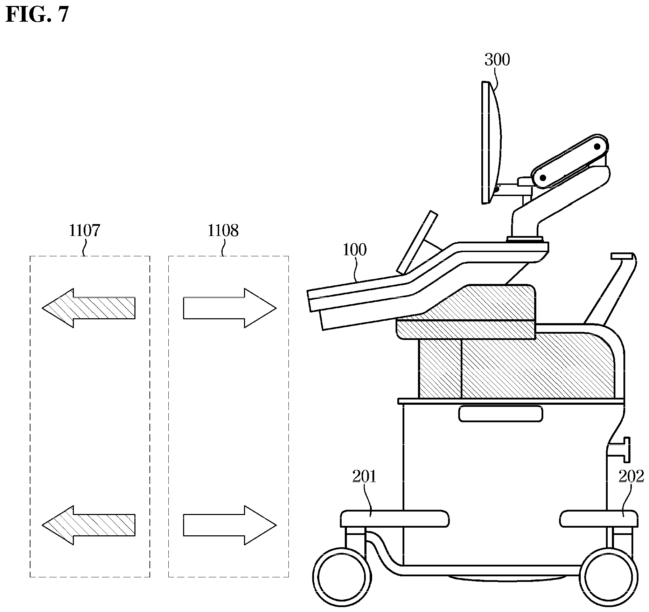

Meanwhile, FIGS. 4 and 5 illustrate and describe that the control panel 100 and the footrest moving device 200 are controlled to the interlocking movement upward and downward. FIGS. 6 and 7 illustrate that the control panel 100 and the footrest moving device 200 are controlled to interlocking movement forward and backward.

FIG. 6 illustrates a third mode of interlocking control of the controller 400. The controller 400 may control the footrest 210 to move backward when the control panel 100 moves forward, and may control the footrest 210 to move forward when the control panel 100 moves backward. The movement of the footrest 210 may be implemented based on the relative movement between the body M, the first support 201 and the second support 202. When the control panel 100 moves forward and the footrest 210 moves backward, the ultrasonic diagnostic apparatus 1 may provide a sufficient space to mount the user's legs.

FIG. 7 illustrates a fourth mode of interlocking control of the controller 400. The controller 400 may control the footrest 210 to move forward when the control panel 100 moves forward, and may control the footrest 210 to move backward when the control panel 100 moves backward. According to an embodiment, the controller 400 may allow the control panel 100 and the footrest 210 to access the user's body side by side at the same time, thereby providing the user's desired settings faster than a conventional independent method.

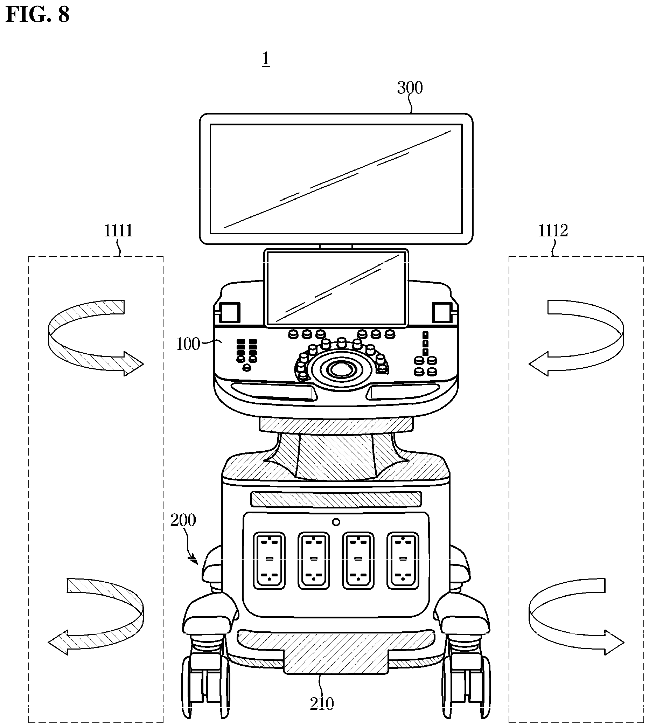

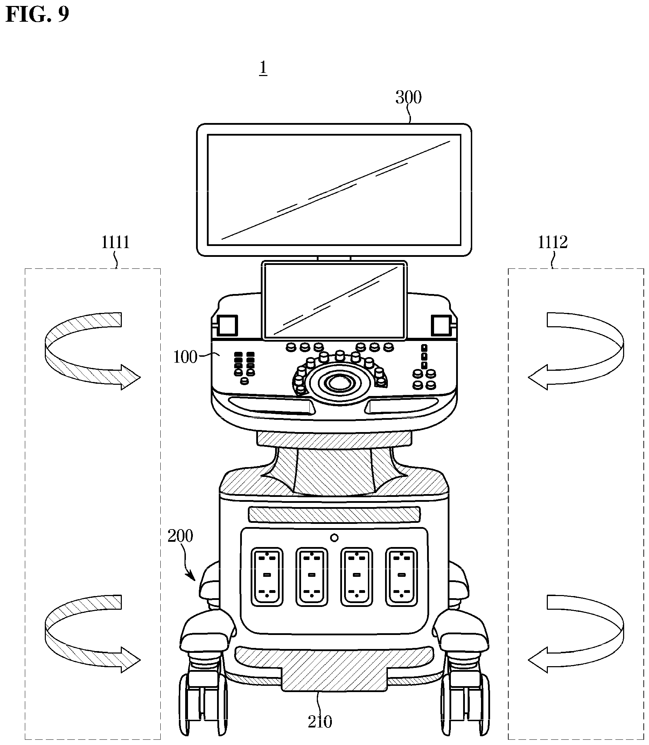

Meanwhile, FIGS. 4 to 7 illustrate that the control panel 100 and the footrest moving device 200 are controlled to interlocking movement upward and downward and forward and backward. FIGS. 8 and 9 illustrate that the control panel 100 and the footrest moving device 200 are controlled to rotate in interlocking with each other, unlike FIGS. 4 to 7.

FIG. 8 illustrates a fifth mode of interlocking control of the controller 400. The controller 400 may control the footrest 210 to rotate clockwise when the control panel 100 rotates counterclockwise, and may control the footrest 210 to rotate counterclockwise when the control panel 100 rotates clockwise. At this time, since the control panel 100 and the footrest 210 rotate in opposite directions to each other, the control panel 100 and the footrest 210 may bring relatively large rotational displacements.

FIG. 9 illustrates a sixth mode of interlocking control of the controller 400. The controller 400 may control the footrest 210 to rotate counterclockwise when the control panel 100 rotates counterclockwise, and may control the footrest 210 to rotate clockwise when the control panel 100 rotates clockwise. When the user is not in front of the ultrasonic diagnostic apparatus 1, the controller 400 may quickly provide the setting desired by the user by rotating the control panel 100 and the footrest 210 together.

Note that other embodiments of the disclosure are not limited to the first to sixth modes described with reference to FIGS. 4 to 9. For example, the ultrasonic diagnostic apparatus 1 according to an embodiment may be interlocked so that the control panel 100 rotates clockwise or counterclockwise and the footrest 210 moves upward or downward. In other words, the controller 400 may adopt various exercise methods according to the user's setting or the user's body structure, and may control the control panel 100 and the footrest 210 together to provide the setting suitable for the user.

In addition, the controller 400 may cooperatively control within the limits of a moving range and a rotating range based on user's body information stored in the memory 402.

According to an aspect of the disclosure as described above, by simultaneously adjusting the control panel and the footrest, the user may quickly provide the desired settings.

Meanwhile, the disclosed exemplary embodiments may be implemented in the form of a recording medium storing instructions that are executable by a computer. The instructions may be stored in the form of a program code, and when executed by a processor, the instructions may generate a program module to perform operations of the disclosed exemplary embodiments. The recording medium may be implemented non-transitory as a computer-readable recording medium.

The non-transitory computer-readable recording medium may include all kinds of recording media storing commands that can be interpreted by a computer. For example, the non-transitory computer-readable recording medium may be, for example, ROM, RAM, a magnetic tape, a magnetic disc, flash memory, an optical data storage device, etc.

Embodiments of the disclosure have thus far been described with reference to the accompanying drawings. It will be obvious to those of ordinary skill in the art that the disclosure may be practiced in other forms than the embodiments as described above without changing the technical idea or essential features of the disclosure. The above embodiments are only by way of example, and should not be interpreted in a limited sense.

## Claims

1. An ultrasonic diagnostic apparatus (1) comprising:
a control panel (100) configured to receive a user input for operating the ultrasonic diagnostic apparatus, and to move a position of the control panel (100) with respect to the ultrasonic diagnostic apparatus;
a footrest moving device (200) provided below the ultrasonic diagnostic apparatus, configured to move a footrest provided to mount a user's foot and the ultrasonic diagnostic apparatus (1) with respect to the ground;
wherein the footrest moving device (200) comprises:
a first support (201) configured to support the ultrasonic apparatus on a lower front of the ultrasonic diagnostic apparatus; and
a second support (202) configured to support the ultrasonic apparatus on a lower rear of the ultrasonic diagnostic apparatus,
**characterized in that** the first and second support are configured to be each changed in height; and
that the ultrasonic diagnostic apparatus further comprises a controller configured to control the movement of the control panel and the footrest moving device to interlock.

2. The ultrasonic diagnostic apparatus (1) according to claim 1, wherein the controller is configured to move the footrest downward when the control panel (100) moves upward, and to move the footrest upward when the control panel moves downward.

3. The ultrasonic diagnostic apparatus (1) according to claim 1, wherein the controller is configured to move the footrest upward when the control panel (100) moves upward, and to move the footrest upward when the control panel moves downward.

4. The ultrasonic diagnostic apparatus (1) according to claim 1, wherein the controller is configured to move the footrest backward when the control panel (100) moves forward, and to move the footrest forward when the control panel (100) moves backward.

5. The ultrasonic diagnostic apparatus (1) according to claim 1, wherein the controller is configured to move the footrest forward when the control panel (100) moves forward, and to move the footrest backward when the control panel moves backward.

6. The ultrasonic diagnostic apparatus (1) according to claim 1, wherein the controller is configured to move the footrest clockwise when the control panel (100) moves counterclockwise, and to move the footrest counterclockwise when the control panel moves clockwise.

7. The ultrasonic diagnostic apparatus (1) according to claim 1, wherein the controller is configured to move the footrest counterclockwise when the control panel (100) moves counterclockwise, and to move the footrest clockwise when the control panel moves clockwise.

8. The ultrasonic diagnostic apparatus (1) according to claim 1, further comprising: a memory (402) configured to store mode settings of the interlocking.

9. The ultrasonic diagnostic apparatus according to claim 1, further comprising:
an inputter (110) configured to receive an input for operating the interlocking of the control panel (100) and the footrest,
wherein the inputter (110) is positioned at any one of a main body of the ultrasonic diagnostic apparatus and the control panel.

10. The ultrasonic diagnostic apparatus (1) according to claim 9, wherein the inputter (110) comprises a mode change button, a first move button (111), and a second move button (112) configured to move in a direction opposite to a movement by the first move button.

11. The ultrasonic diagnostic apparatus (1) according to claim 1, wherein the control panel (100) is configured to be changed in position by upward and downward movement, left and right movement, forward and backward movement, rotational movement, and tilting.

12. The ultrasonic diagnostic apparatus (1) according to claim 1,
wherein the footrest moving device (200) is configured to be changed in position by upward and downward movement, left and right movement, forward and backward movement, rotational movement, and tilting, and
wherein the tilting is performed by upward and downward movement of the first support (201).

## Patentansprüche

1. Ultraschalldiagnosevorrichtung (1) mit:
einem Bedienfeld (100), das dazu ausgelegt ist, eine Benutzereingabe zum Betreiben der Ultraschalldiagnosevorrichtung zu empfangen, und eine Position des Bedienfelds (100) relativ zur Ultraschalldiagnosevorrichtung zu ändern;
einer Fußstützenbewegungsvorrichtung (200), die unter der Ultraschalldiagnosevorrichtung vorgesehen und dazu ausgelegt ist, eine den Fuß eines Benutzers aufzunehmende Fußstütze und die Ultraschalldiagnosevorrichtung (1) relativ zum Boden zu verstellen;
wobei die Fußstützenbewegungsvorrichtung (200) Folgendes umfasst:
einen ersten Träger (201), der dazu ausgelegt ist, eine Unterseite der Ultraschallvorrichtung an einer Frontseite der Ultraschalldiagnosevorrichtung zu tragen, und
einen zweiten Träger (202), der dazu ausgelegt ist, eine Unterseite der Ultraschallvorrichtung an einer Rückseite der Ultraschalldiagnosevorrichtung zu tragen,
**dadurch gekennzeichnet, dass** der erste und der zweite Träger so ausgelegt sind, dass sie jeweils höhenverstellbar sind, und
dadurch, dass die Ultraschalldiagnosevorrichtung ferner eine Steuerung umfasst, die dazu ausgelegt ist, das Verstellen des Bedienfelds und der Fußstützenbewegungsvorrichtung in verriegelter Weise zu steuern.

2. Ultraschalldiagnosevorrichtung (1) nach Anspruch 1, wobei die Steuerung dazu ausgelegt ist, die Fußstütze nach unten zu verstellen, wenn das Bedienfeld (100) nach oben verstellt wird, und die Fußstütze nach oben zu verstellen, wenn das Bedienfeld nach unten verstellt wird.

3. Ultraschalldiagnosevorrichtung (1) nach Anspruch 1, wobei die Steuerung dazu ausgelegt ist, die Fußstütze nach oben zu verstellen, wenn das Bedienfeld (100) nach oben verstellt wird, und die Fußstütze nach oben zu verstellen, wenn das Bedienfeld nach unten verstellt wird.

4. Ultraschalldiagnosevorrichtung (1) nach Anspruch 1, wobei die Steuerung dazu ausgelegt ist, die Fußstütze rückwärts zu verstellen, wenn das Bedienfeld (100) vorwärts verstellt wird, und die Fußstütze vorwärts zu verstellen, wenn das Bedienfeld (100) rückwärts verstellt wird.

5. Ultraschalldiagnosevorrichtung (1) nach Anspruch 1, wobei die Steuerung dazu ausgelegt ist, die Fußstütze vorwärts zu verstellen, wenn das Bedienfeld (100) vorwärts verstellt wird, und die Fußstütze rückwärts zu verstellen, wenn das Bedienfeld rückwärts verstellt wird.

6. Ultraschalldiagnosevorrichtung (1) nach Anspruch 1, wobei die Steuerung dazu ausgelegt ist, die Fußstütze im Uhrzeigersinn zu verstellen, wenn das Bedienfeld (100) entgegen dem Uhrzeigersinn verstellt wird, und die Fußstütze entgegen dem Uhrzeigersinn zu verstellen, wenn das Bedienfeld im Uhrzeigersinn verstellt wird.

7. Ultraschalldiagnosevorrichtung (1) nach Anspruch 1, wobei die Steuerung dazu ausgelegt ist, die Fußstütze entgegen dem Uhrzeigersinn zu verstellen, wenn das Bedienfeld (100) entgegen dem Uhrzeigersinn verstellt wird, und die Fußstütze im Uhrzeigersinn zu verstellen, wenn das Bedienfeld im Uhrzeigersinn verstellt wird.

8. Ultraschalldiagnosevorrichtung (1) nach Anspruch 1, ferner mit:
einem Speicher (402), der zum Speichern von Moduseinstellungen des Verriegelns ausgelegt ist.

9. Ultraschalldiagnosevorrichtung nach Anspruch 1, ferner mit:
eine Eingabeeinheit (110), die dazu ausgelegt ist, eine Eingabe zum Betreiben des Verriegelns des Bedienfelds (100) und der Fußstütze zu empfangen,
wobei die Eingabeeinheit (110) an einem Hauptkörper der Ultraschalldiagnosevorrichtung oder an dem Bedienfeld positioniert ist.

10. Ultraschalldiagnosevorrichtung (1) nach Anspruch 9, wobei die Eingabeeinheit (110) eine Moduswechseltaste, eine erste Bewegungstaste (111) und eine zweite Bewegungstaste (112) umfasst, welche dazu ausgelegt ist, ein Verstellen in eine Richtung entgegengesetzt zu einem durch die erste Bewegungstaste verursachten Verstellen zu ermöglichen.

11. Ultraschalldiagnosevorrichtung (1) nach Anspruch 1, wobei das Bedienfeld (100) so ausgelegt ist, dass seine Position durch Aufwärts- und Abwärtsbewegung, Links- und Rechtsbewegung, Vorwärts- und Rückwärtsbewegung, Drehbewegung und Kippen verändert werden kann.

12. Ultraschalldiagnosevorrichtung (1) nach Anspruch 1,
wobei die Fußstützenbewegungsvorrichtung (200) so ausgelegt ist, dass seine Position durch Aufwärts- und Abwärtsbewegung, Links- und Rechtsbewegung, Vorwärts- und Rückwärtsbewegung, Drehbewegung und Kippen verändert werden kann, und
wobei das Kippen durch Aufwärts- und Abwärtsbewegung des ersten Trägers (201) erfolgt.

## Revendications

1. Appareil d'échographie diagnostique (1) comprenant :
un tableau de commande (100) conçu pour recevoir une entrée utilisateur permettant d'exploiter l'appareil d'échographie diagnostique, et pour faire changer le tableau de commande (100) de position par rapport à l'appareil d'échographie diagnostique ;
un dispositif d'adaptation de repose-pied (200) prévu sous l'appareil d'échographie diagnostique et conçu pour adapter la position d'un repose-pied, prévu pour recevoir le pied d'un utilisateur, et de l'appareil d'échographie diagnostique (1) par rapport au sol ;
ledit dispositif d'adaptation de repose-pied (200) comprenant :
un premier support (201) conçu pour supporter le bas de l'appareil d'échographie, sur l'avant de l'appareil d'échographie diagnostique, et
un deuxième support (202) conçu pour supporter le bas de l'appareil d'échographie, sur l'arrière de l'appareil d'échographie diagnostique ;
**caractérisé en ce que** les premier et deuxième supports sont conçus de façon à pouvoir chacun changer de hauteur, et
**en ce que** l'appareil d'échographie diagnostique comprend en outre un organe de commande conçu pour commander l'adaptation de position du tableau de commande et du dispositif d'adaptation de repose-pied de façon qu'ils soient interverrouillés.

2. Appareil d'échographie diagnostique (1) selon la revendication 1, dans lequel l'organe de commande est conçu pour faire descendre le repose-pied lorsque le tableau de commande (100) monte, et pour faire monter le repose-pied lorsque le tableau de commande descend.

3. Appareil d'échographie diagnostique (1) selon la revendication 1, dans lequel l'organe de commande est conçu pour faire monter le repose-pied lorsque le tableau de commande (100) monte, et pour faire monter le repose-pied lorsque le tableau de commande descend.

4. Appareil d'échographie diagnostique (1) selon la revendication 1, dans lequel l'organe de commande est conçu pour faire reculer le repose-pied lorsque le tableau de commande (100) avance, et pour faire avancer le repose-pied lorsque le tableau de commande (100) recule.

5. Appareil d'échographie diagnostique (1) selon la revendication 1, dans lequel l'organe de commande est conçu pour faire avancer le repose-pied lorsque le tableau de commande (100) avance, et pour faire reculer le repose-pied lorsque le tableau de commande recule.

6. Appareil d'échographie diagnostique (1) selon la revendication 1, dans lequel l'organe de commande est conçu pour faire tourner le repose-pied dans le sens des aiguilles d'une montre lorsque le tableau de commande (100) tourne dans le sens inverse des aiguilles d'une montre, et pour faire tourner le repose-pied dans le sens inverse des aiguilles d'une montre lorsque le tableau de commande tourne dans le sens des aiguilles d'une montre.

7. Appareil d'échographie diagnostique (1) selon la revendication 1, dans lequel l'organe de commande est conçu pour faire tourner le repose-pied dans le sens inverse des aiguilles d'une montre lorsque le tableau de commande (100) tourne dans le sens inverse des aiguilles d'une montre, et pour faire tourner le repose-pied dans le sens des aiguilles d'une montre lorsque le tableau de commande tourne dans le sens des aiguilles d'une montre.

8. Appareil d'échographie diagnostique (1) selon la revendication 1, comprenant en outre : une mémoire (402) conçue pour contenir des paramètres de mode d'interverrouillage.

9. Appareil d'échographie diagnostique selon la revendication 1, comprenant en outre :
un dispositif d'entrée (110) conçu pour recevoir une entrée permettant d'exploiter l'interverrouillage du tableau de commande (100) et du repose-pied ;
ledit dispositif d'entrée (110) étant disposé sur un corps principal de l'appareil d'échographie diagnostique ou bien sur le tableau de commande.

10. Appareil d'échographie diagnostique (1) selon la revendication 9, dans lequel le dispositif d'entrée (110) comprend un bouton de changement de mode, un premier bouton d'adaptation (111) et un deuxième bouton d'adaptation (112) conçu pour permettre une adaptation de position dans une direction opposée à une adaptation de position permise par le premier bouton d'adaptation.

11. Appareil d'échographie diagnostique (1) selon la revendication 1, dans lequel le tableau de commande (100) est conçu pour changer de position selon un déplacement ascendant et descendant, un déplacement vers la gauche et vers la droite, un déplacement d'avancée et de recul, un déplacement en rotation et un pivotement.

12. Appareil d'échographie diagnostique (1) selon la revendication 1,
dans lequel le dispositif d'adaptation de repose-pied (200) est conçu pour changer de position selon un déplacement ascendant et descendant, un déplacement vers la gauche et vers la droite, un déplacement d'avancée et de recul, un déplacement en rotation et un pivotement, et
dans lequel le pivotement est réalisé sous l'effet d'un déplacement ascendant et d'un déplacement descendant du premier support (201).
